(19) Europäisches Patentamt
European Patent Office
Office européen des brevets

(11) Veröffentlichungsnummer: **0 194 525**
**B1**

(12) **EUROPÄISCHE PATENTSCHRIFT**

(45) Veröffentlichungstag der Patentschrift: **10.08.88**

(51) Int. Cl.⁴: **C 07 D 233/56,** C 07 D 249/08, A 01 N 47/38

(21) Anmeldenummer: **86102623.5**

(22) Anmeldetag: **28.02.86**

(54) **N-Carbamoyl-azole enthaltende Fungizide.**

(30) Priorität: **05.03.85 DE 3507630**

(43) Veröffentlichungstag der Anmeldung: **17.09.86 Patentblatt 86/38**

(45) Bekanntmachung des Hinweises auf die Patenterteilung: **10.08.88 Patentblatt 88/32**

(84) Benannte Vertragsstaaten: **AT BE CH DE FR GB IT LI NL SE**

(56) Entgegenhaltungen: **EP-A-0 175 188**
**FR-A-2 234 293**

(73) Patentinhaber: **BASF Aktiengesellschaft, Carl-Bosch- Strasse 38, D-6700 Ludwigshafen (DE)**

(72) Erfinder: **Karbach, Stefan, Dr., Sperlinggasse 3, D-6700 Ludwigshafen (DE)**
Erfinder: **Rentzea, Costin, Dr., Richard- Kuhn-Strasse 1-3, D-6900 Heidelberg (DE)**
Erfinder: **Ammermann, Eberhard, Dr., Sachsenstrasse 3, D-6700 Ludwigshafen (DE)**
Erfinder: **Pommer, Ernst- Heinrich, Dr., Berliner Platz 7, D-6703 Limburgerhof (DE)**

**EP 0 194 525 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue N-Carbamoyl-azole, Verfahren zu ihrer Herstellung und fungizide Mittel, die diese Verbindungen als Wirkstoff enthalten.

Es ist bekannt, N-Isopropyl-N-phenyloxiethyl-N'-imidazolylharnstoff als Fungizid zu verwenden (GB-1 469 772). Seine Wirkung genügt bei niedrigen Aufwandmengen den Anforderungen der Praxis nicht.

Es wurde nun gefunden, daß N-Carbamoyl-azole der Formel

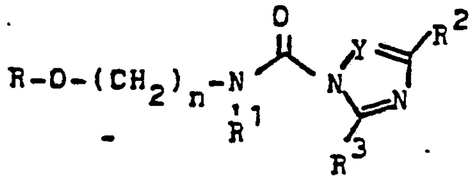

(I),

in der

R einen gegebenenfalls alkyl-, alkoxyalkyl-, alkenyl-, cycloalkylsubstituierten Cycloalkylrest oder Bicycloalkylrest oder einen mono- oder bicyclischen Terpenylrest bedeutet,

n = 4, 5 oder 6,

$R^1$ = Alkyl, Alkenyl, Alkoxyalkyl, Alkylthioalkyl, Cycloalkyl, Cycloalkylalkyl, Alkoxy, Alkenoxy, Alkoxyalkoxy oder einen gegebenenfalls substituierten Benzylrest bedeutet, und

Y = CH, $C-CH_3$ oder N bedeutet, und

$R^2$ und $R^3$ gleich oder verschieden sind und Wasserstoff oder Alkyl mit 1 - 5 C-Atomen bedeuten, sehr gut wirksam gegen Schadpilze sind.

Einige neue Verbindungen der Formel I enthalten chirale Zentren und werden im allgemeinen in Form von Mischungen (Racemate oder Diasteromerengemische) erhalten. Die Diastereomeren lassen sich bei einigen der erfindungsgemäßen Verbindungen beispielsweise durch Säulenchromatographie trennen oder aufgrund von Löslichkeitsunterschieden in reiner Form isolieren. Aus solchen Diastereomeren kann man mit bekannten Methoden einheitliche Enantiomere erhalten. Diese werden wie ihre Mischungen von der vorliegenden Erfindung umfaßt. Für die Anwendung der neuen Verbindungen als Fungizide sind sowohl die Diastereomeren bzw. Enantiomeren wie auch deren bei der Synthese anfallenden Gemische geeignet. Bevorzugt werden die letzteren verwendet.

Als Bedeutung für R in Formel I kommen Reste z. B. $C_5$-$C_{12}$-Cycloalkyl, wie Cyclopentyl, Cyclohexyl, Cycloheptyl, Cyclooctyl, Cyclodecyl, Cyclododecyl, durch $C_1$-$C_9$-Alkyl substituiertes $C_5$-$C_8$-Cycloalkyl, 4-Nonylcyclohexyl, 4-Hexylcyclohexyl, 4-Butylcyclohexyl, 4-tert.-Butylcyclohexyl, durch Cyclohexyl substituiertes Cyclohexyl, 4-Cyclohexylcyclohexyl, 2-Cy-clohexylcyclohexyl, 3-Cyclohexylcyclohexyl, gegebenenfalls durch $C_1$-$C_4$-Alkyl substituiertes $C_8$-$C_9$-Bicycloalkyl, 1,5-Dimethylbicyclo[2.3.1]octyl, 3.3.5-Trimethylcyclohexyl, 2,4-Dimethylcyclohexyl, 9-Ethyl-bicyclo-[3.3.1]non-9-yl, $C_{10}$-Terpenyl, Norbornyl, Menthyl, Bornyl, Isobornyl, Carvenyl, Pinanyl, Caranyl; 5-Norbonen-2-yl, Verbenyl, Isopinocamphenyl, 1-Adamantyl, 2-Adamantyl,

für $R^1$ kommen Reste wie Ethyl, n-Propyl, Isopropyl, n-Butyl, Isobutyl, sek.-Butyl, tert.-Butyl, n-Pentyl, 3-Methyl-1-butyl, n-Hexyl, 3,3-Dimethylbutyl-1, n-Heptyl, n-Octyl, 2,4,4-Trimethylpentyl, 2-Ethylhexyl, n-Decyl, n-Dodecyl, Cyclopropylmethyl, Cyclopentyl, Cyclohexyl, 4-Methylcyclohexyl, Cyclohexylmethyl, 4-ter.-Butylcyclohexyl, 2-Methoxyethyl, 2-Ethoxyethyl, 2-Propoxyethyl, 2-Butoxyethyl, 3-Methoxypropyl, 3-Ethoxypropyl, 3-Propoxypropyl, 3-Butoxypropyl, Allyl, 2-Buten-1-yl, 2-Penten-1yl, 2-Octen-1-yl, Benzyl, 4-Methylbenzyl, alpha-Methylbenzyl, alpha-Ethylbenzyl, 2-, 3- und 4-Fluorbenzyl, 2-, 3- und 4-Chlorbenzyl, 4-Brombenzyl, 3- und 4-Trifluormethylbenzyl, 4-tert.-Butylbenzyl, p-Methoxybenzyl, 2,4-Dichlorbenzyl, Phenylethyl, Ethoxy, n-Propyloxy, Isopropyloxy, n-Butyloxy, n-Hexyloxy, Cyclohexyloxy, 4-Cyclohexyloxybutyloxy, 2-Ethoxyethyloxy

in Betracht,

n bedeutet 4, 5 oder 6

Y = CH, N oder $C-CH_3$

$R^2$ und $R^3$ bedeuten vorzugsweise Wasserstoff, Methyl, Ethyl, n-Propyl und Isopropyl.

Die Verbindungen der Formel I lassen sich herstellen, indem man ein Carbamoylchlorid der Formel II

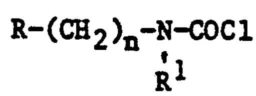

(II),

in der R und $R^1$ die oben angegebenen Bedeutungen haben,

a) mit Azolen der Formel III

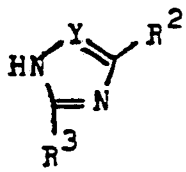 (III),

in der $R^2$, $R^3$ und Y die oben angegebenen Bedeutungen haben, oder

b) mit deren Metall-Derivaten der Formel IV

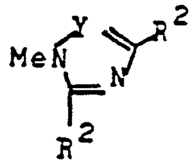 (IV),

in der $R^2$, $R^3$ und Y die oben angegebenen Bedeutungen haben und Me Lithium, Natrium, Kalium oder 1/2 Calcium bedeutet, oder

c) mit deren Silyl-Derivaten der Formel V ££30 in der $R^2$, $R^3$ und Y die oben angegebenen Bedeutungen haben, umsetzt.

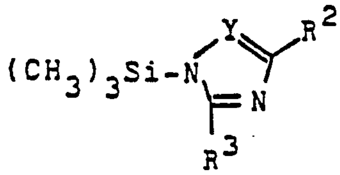 (V),

Die Reaktion a) erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsgeschleunigers bei Temperaturen zwischen 10 und 120°C.

Als bevorzugte, gegenüber den Reaktionsteilnehmern, inerte Lösungs- oder Verdünnungsmittel können beispielsweise aliphatische oder aromatische, gegebenenfalls halogenierte Kohlenwasserstoffe, wie n-Pentan, Cyclohexan, Methylenchlorid, 1,1,1-Trichlorethan, Benzol, Toluol, Xylol, Chlorbenzol, aliphatische Ketone, wie Aceton, Methylethylketon, Diethylketon, Ether, wie Diethylether, Methyl-tert.-butylether, Dimethoxyethan, Tetrahydrofuran oder Dioxan, Ester, wie Essigsäureethylester, Nitrile wie Acetonitril, Amide, wie Dimethylformamid, Dimethylacetamid und N-Methylpyrrolidon oder Gemische dieser Lösungsmittel verwendet werden.

Geeignete Basen, die gegebenenfalls auch als säurebindende Mittel bei der Reaktion verwendet werden können, sind beispielsweise Alkalihydroxide wie Lithium, Natrium-, Kalium- oder Calciumhydroxid, Alkalicarbonate wie Natrium- oder Kaliumcarbonat oder Natrium- und Kaliumhydrogencarbonat, Amine wie Triethylamin, Tripropylamin, N-Methylpyrrolidin, N-Methylpiperidin, N,N-Dimethylcyclohexylamin, N,N'-Tetramethylethylendiamin, N,N-Dimethylanilin, N,N-Diethylanilin, Pyridin oder 4-Dimethylaminopyridin. Es können aber auch andere übliche Basen verwendet werden.

Als Reaktionsbeschleuniger kommen vorzugsweise Metallhalogenide wie 5 Natriumiodid oder Kaliumiodid, quaternäre Ammoniumsalze wie Tetrabutylammonium-chlorid, -bromid, -iodid und -hydrogensulfat, Benzyltriethylammonium-chlorid oder -bromid oder Kronenether wie 12-Krone-4, 15-Krone-5, 18-Krone-6 oder Dibenzo-18-krone-6 in Frage.

Die Reaktionen b) und c) erfolgen gegebenenfalls in Gegenwart eines Lösungs- oder Verdünnungsmittels bei einer Temperatur im Bereich zwischen 0 und 140°C, vorzugsweise zwischen 0 und 100°C. Hierfür eignen sich dieselben Lösungsmittel wie für die Verfahrensvariante a).

Die Verbindung der Formel I lassen sich ferner herstellen, indem man eine Verbindung der Formel VI

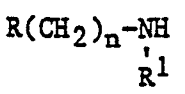 (VI),

in der R, $R^1$ und n die oben angegebenen Bedeutungen haben, mit einem Carbonyl-bisazol der Formel VII

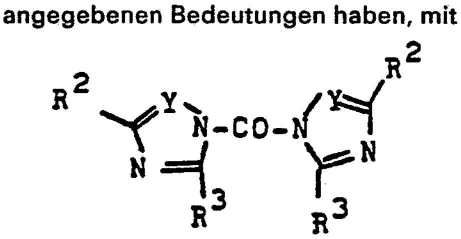 (VII),

in der $R^2$, $R^3$ und Y die oben angegebenen Bedeutungen haben, gegebenenfalls in Gegenwart eines Lösungs- oder Verdünnungsmittels, gegebenenfalls unter Zusatz eines Reaktionsbeschleunigers, umsetzt.

Hierfür eignen sich beispielsweise folgende Lösungs- oder Verdünnungsmittel: Diethylether, 1,2-Dimethoxyethan, Dipropylether, Dibutylether, Methyl-tert.-butylether, Tetrahydrofuran, Dimethoxyethan,

Anisol, n-Pentan, n-Hexan, n-Heptan, n-Octan, Isooctan, Cyclohexan, Toluol, Chlorbenzol, Xylole, Acetonitril, Essigester, Dimethylformamid, N-Methylpyrrolidon, Aceton oder Methylketon.

Geeignete Reaktionsbeschleuniger sind beispielsweise 4-Dimethylaminopyridin oder 4-Pyrrolidinopyridin.

Die Ausgangsstoffe der Formel II können leicht nach bekannten Verfahren hergestellt werden, beispielsweise durch Umsetzung von Aminen der Formel VI mit Phosgen (Houben-Weyl-Müller, Methoden oder organischen Chemie, Band 8, Seite 115 bis 118, Georg Thieme Verlag, Stuttgart, 1952).

Die folgenden Beispiele erläutern die Herstellung der Verbindungen der Formel I.

**Beispiel**

a) 57,3 g (0,3 Mol) 4-Cyclohexyloxibutylchlorid werden in 200 ml n-Propylamin unter Rückfluß 24 Stunden gerührt. Das Reaktionsgemisch wird unter vermindertem Druck eingeengt, der Rückstand in 400 ml 3 N HCl gelöst und mit 2 x 75 ml Toluol extrahiert. Die wässerige Phase wird mit konz. Natronlauge stark alkalisch gestellt und das sich abscheidende Öl mit Ether extrahiert. Nach Einengen der organischen Phase verbleiben 46,7 g N-(Cyclohexyloxibutyl)-N-propylamin.

b) In 21 ml einer 25 %-igen Phosgen-Lösung in Toluol werden bei 0°C 8,95 g N-(n-Cyclohexyloxibutyl)-N-propylamin in 70 ml trockenem Toluol und 4 g Pyridin zugetropft. Nach Zugabe der Reaktanden wird auf Raumtemperatur und anschließend 1 h auf 100°C erwärmt. Nach Abkühlen des Reaktionsgemisches wird Eis zugegeben und 1 x mit 25 ml 5 %-iger $KHSO_4$-Lösung, 2 x mit 3 %-iger $Na_2CO_3$ und 1 x mit Wasser ausgeschüttelt. Nach Trocknung der organischen Phase über Natriumsulfat wird unter vermindertem Druck das Lösungsmittel abgezogen. Es bleiben 11,57 g reines N-(4-Cyclohexyloxibutyl)-N-propylcarbamoylchlorid zurück.

c) 5,7 g Natriumtriazolid werden in 30 ml trockenem Tetrahydrofuran gelöst und 11,5 g N-(4-Cyclohexyloxibutyl)-N-propylcarbamoylchlorid in 30 ml Tetrahydrofuran unter Rühren zugetropft. Nach Zugabe des Carbamoylchlorids wird 3 Stunden unter Rückfluß sieden gelassen. Es wird noch 15 Stunden bei Raumtemperatur nachgerührt. Das Reaktionsgemisch wird anschließend unter vermindertem Druck eingeengt und in 100 ml Methylenchlorid gelöst. Nach Waschen mit 3 %-iger Natriumcarbonat-Lösung und Wasser wird über Natriumsulfat getrocknet und das Lösungsmittel unter vermindertem Druck abdestilliert. Es bleiben 9,2 g N-(Cyclohexyloxibutyl)-N-propylcarbamoyltriazol als hellgelbes Öl zurück. (Verbindung Nr. 1)

Die nachstehend in der Tabelle aufgeführten Verbindungen wurden auf dem angegebenen Weg erhalten; andere Verbindungen, die der Formel I entsprechen, können auf die gleich Weise unter entsprechender Änderung der Mengenangaben der Vorschriften erhalten werden.

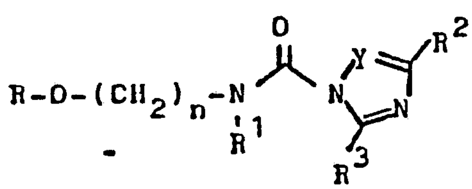

| Verbindung Nr. | R | n | $R^1$ | $R^2$ | $R^3$ | Y | IR: |
|---|---|---|---|---|---|---|---|
| 1 | Cyclohexyl | 4 | n-$C_3H_7$ | H | H | N | 2932, 2856, 1701, 1466, 1450, 1429, 1381, 1276, 1138, 1106 |
| 2 | Cyclohexyl | 4 | n-$C_3H_7$ | H | H | CH | 2932, 2856, 1694, 1465, 1451, 1422, 1275, 1238, 1101 |
| 3 | Cyclohexyl | 4 | n-$C_6H_{13}$ | H | H | N | 2932, 2856, 1702, 1467, 1427, 1378, 1276, 1108 |
| 4 | Cyclohexyl | 4 | n-$C_6H_{13}$ | H | H | CH | 2931, 2856, 1695, 1466, 1452, 1422, 1302, 1276, 1101 |
| 5 | Cyclopentyl | 4 | n-$C_3H_7$ | H | H | N | 2960, 2872, 1696, 1465, 1427, 1348, 1276, 1099 |
| 6 | Cyclopentyl | 4 | n-$C_3H_7$ | H | H | CH | 2960, 2871, 1694, 1465, 1422, 1276, 1238, 1099 |

| | | | | | | | |
|---|---|---|---|---|---|---|---|
| 7 | Cyclopentyl | 4 | n-$C_6H_{13}$ | H | H | CH | 2955, 2869, 1694, 1466, 1422, 1302, 1275, 1231, 1100 |
| 8 | Cycloheptyl | 4 | n-$C_3H_7$ | H | H | CH | 2930, 2857, 1694, 1461, 1422, 1302, 1275, 1238, 1098 |
| 9 | Cycloheptyl | 4 | n-$C_3H_7$ | H | H | N | 2928, 2857, 1700, 1463, 1428, 1381, 1275, 1096, 1000 |
| 10 | Cycloheptyl | 4 | n-$C_6H_{13}$ | H | H | CH | 2928, 2857, 1695, 1461, 1423, 1370, 1302, 1277, 1231, 1099 |
| 11 | Cycloheptyl | 4 | n-$C_6H_{13}$ | H | H | N | 2928, 2857, 1701, 1467, 1428, 1379, 1276, 1203, 1097, 995 |
| 12 | Norbornyl | 4 | n-$C_3H_7$ | H | H | CH | 2955, 2871, 1694, 1465, 1422, 1302, 1276, 1238, 1101 |
| 13 | Norbornyl | 4 | n-$C_3H_7$ | H | H | N | 2956, 2871, 1700, 1468, 1428, 1381, 1358, 1276, 1102 |
| 14 | Norbornyl | 4 | n-$C_6H_{13}$ | H | H | CH | 2954, 2870, 1696, 1466, 1422, 1302, 1277, 1231, 1101 |
| 15 | Norbornyl | 4 | n-$C_6H_{13}$ | H | H | N | 2955, 2870, 1701, 1468, 1454, 1428, 1379, 1276, 1203, 1102 |
| 16 | Norbornyl | 6 | n-$C_3H_7$ | H | H | CH | 2949, 2869, 1694, 1464, 1422, 1303, 1275, 1241, 1100 |
| 17 | Norbornyl | 6 | n-$C_6H_{13}$ | H | H | CH | 2952, 2935, 2868, 1695, 1466, 1422, 1302, 1273, 1232, 1100 |
| 18 | Norbornyl | 6 | n-$C_3H_7$ | H | H | N | 2952, 2869, 1701, 1466, 1428, 1381, 1275, 1100 |
| 19 | Norbornyl | 6 | n-$C_6H_{13}$ | H | H | N | 2953, 2935, 2868, 1701, 1468, 1428, 1379, 1275, 1101 |
| 20 | 3,3,5-Trimethyl-cyclohexyl | 4 | n-$C_6H_{13}$ | H | H | CH | 2951, 2926, 2867, 1695, 1460, 1423, 1366, 1277, 1231, 1102 |
| 21 | 3,3,5-Trimethyl-cyclohexyl | 4 | n-$C_6H_{13}$ | H | H | N | 2951, 2926, 2866, 1701, 1468, 1466, 1428, 1378, 1276, 1104 |

In entsprechender Weise können in der folgenden Tabelle aufgeführte Verbindungen hergestellt werden:

| | | | | | | |
|---|---|---|---|---|---|---|
| 22 | Cyclopentyl | 4 | n-$C_6H_{13}$ | H | H | N |
| 23 | Cyclopentyl | 4 | Cyclohexyl | H | H | CH |
| 24 | Cyclopentyl | 5 | n-$C_6H_{13}$ | H | H | CH |
| 25 | Cyclopentyl | 5 | n-$C_3H_7$ | H | H | CH |
| 26 | Cyclopentyl | 5 | n-$C_3H_7$ | H | H | N |
| 27 | Cyclopentyl | 6 | n-$C_6H_{13}$ | H | H | N |
| 28 | Cyclopentyl | 6 | n-$C_3H_7$ | H | H | CH |
| 29 | Cyclopentyl | 6 | n-Cyclohexyl | H | H | CH |
| 30 | Cyclohexyl | 4 | n-$C_6H_{13}$ | H | iso-$C_3H_7$ | CH |
| 31 | Cyclohexyl | 4 | n-$C_3H_7$ | H | iso-$C_3H_7$ | CH |
| 32 | Cyclohexyl | 4 | n-$C_8H_{17}$ | H | H | CH |
| 33 | Cyclohexyl | 4 | n-$C_8H_{17}$ | H | H | N |
| 34 | Cyclohexyl | 5 | n-$C_3H_7$ | H | H | CH |
| 35 | Cyclohexyl | 5 | n-$C_6H_{13}$ | H | H | CH |
| 36 | Cyclohexyl | 5 | n-$C_6H_{13}$ | H | H | N |
| 37 | Cyclohexyl | 5 | n-$C_6H_{13}$ | H | iso-$C_3H_7$ | CH |
| 38 | Cyclohexyl | 6 | n-$C_3H_7$ | H | H | CH |
| 39 | Cyclohexyl | 6 | n-$C_3H_7$ | H | H | CH |
| 40 | Cyclohexyl | 6 | n-$C_6H_{13}$ | H | H | CH |
| 41 | Cyclohexyl | 6 | n-$C_8H_{17}$ | H | H | N |
| 42 | Cyclohexyl | 6 | Cyclohexyl | H | H | CH |

| | | | | | | |
|---|---|---|---|---|---|---|
| 43 | Cycloheptyl | 4 | n-Cyclohexyl | H | H | CH |
| 44 | Cycloheptyl | 4 | n-Cyclohexyl | H | H | N |
| 45 | Cycloheptyl | 4 | n-$C_6H_{13}$ | H | iso-$C_3H_7$ | CH |
| 46 | Cycloheptyl | 5 | n-$C_3H_7$ | H | H | CH |
| 47 | Cycloheptyl | 5 | n-$C_6H_{13}$ | H | H | CH |
| 48 | Cycloheptyl | 5 | Cyclohexyl | H | H | CH |
| 49 | Cycloheptyl | 5 | Cyclohexyl | H | iso-$C_3H_7$ | CH |
| 50 | Cycloheptyl | 5 | Cyclohexyl | H | H | N |
| 51 | Cycloheptyl | 5 | n-$C_3H_7$ | H | | N |
| 52 | Cycloheptyl | 5 | n-$C_6H_{13}$ | H | iso-$C_3H_7$ | CH |
| 53 | Cycloheptyl | 5 | n-$C_6H_{13}$ | H | | N |
| 54 | Cyclooctyl | 4 | n-$C_3H_7$ | H | H | CH |
| 55 | Cyclooctyl | 4 | n-$C_3H_7$ | H | H | N |
| 56 | Cyclooctyl | 4 | n-$C_3H_7$ | H | iso-$C_3H_7$ | CH |
| 57 | Cyclooctyl | 4 | n-$C_6H_{13}$ | H | H | CH |
| 58 | Cyclooctyl | 4 | n-$C_6H_{13}$ | H | H | N |
| 59 | Cyclooctyl | 4 | n-$C_6H_{13}$ | H | iso-$C_3H_7$ | CH |
| 60 | Cyclooctyl | 4 | Cyclohexyl | H | H | CH |
| 61 | Cyclooctyl | 5 | n-$C_3H_7$ | H | H | CH |
| 62 | Cyclooctyl | 5 | n-$C_3H_7$ | H | H | N |
| 63 | Cyclooctyl | 5 | n-$C_3H_7$ | H | iso-$C_3H_7$ | CH |
| 64 | Cyclooctyl | 5 | n-$C_6H_{13}$ | H | H | CH |
| 65 | Cyclooctyl | 5 | n-$C_6H_{13}$ | H | H | N |
| 66 | Cyclooctyl | 5 | n-$C_6H_{13}$ | H | iso-$C_3H_7$ | CH |
| 67 | Cyclododecyl | 4 | n-$C_3H_7$ | H | H | CH |
| 68 | Cyclododecyl | 4 | n-$C_3H_7$ | H | H | N |
| 69 | Cyclododecyl | 4 | n-$C_3H_7$ | H | iso-$C_3H_7$ | CH |
| 70 | Cyclododecyl | 4 | n-$C_6H_{13}$ | H | H | CH |
| 71 | Cyclododecyl | 4 | n-$C_6H_{13}$ | H | H | N |
| 72 | Cyclododecyl | 4 | n-$C_6H_{13}$ | H | iso-$C_3H_7$ | CH |
| 73 | Cyclododecyl | 4 | Cyclohexyl | H | H | CH |
| 74 | Cyclododecyl | 4 | Cyclohexyl | H | H | N |
| 75 | Cyclododecyl | 4 | Cyclohexyl | H | iso-$C_3H_7$ | CH |
| 76 | Cyclodedecyl | 4 | Cyclohexyl | $CH_3$ | $CH_3$ | CH |
| 77 | Cyclododecyl | 5 | n-$C_3H_7$ | H | H | CH |
| 78 | Cyclododecyl | 5 | n-$C_3H_7$ | H | H | N |
| 79 | Cyclododecyl | 5 | n-$C_3H_7$ | H | iso-$C_3H_7$ | CH |
| 80 | Cyclododecyl | 5 | n-$C_6H_{13}$ | H | H | CH |
| 81 | Cyclododecyl | 5 | n-$C_6H_{13}$ | H | H | N |
| 82 | Cyclododecyl | 5 | n-$C_6H_{13}$ | H | iso-$C_3H_7$ | CH |
| 83 | Cyclododecyl | 5 | Cyclohexyl | H | H | CH |
| 84 | Cyclododecyl | 5 | Cyclohexyl | H | H | N |
| 85 | Cyclododecyl | 5 | Cyclohexyl | H | iso-$C_3H_7$ | CH |
| 86 | 4-Nonylcyclohexyl | 4 | n-$C_3H_7$ | H | H | CH |
| 87 | 4-Nonylcyclohexyl | 4 | n-$C_3H_7$ | H | H | N |
| 88 | 4-Nonylcyclohexyl | 4 | n-$C_6H_{13}$ | H | H | CH |
| 89 | 4-Nonylcyclohexyl | 4 | n-$C_6H_{13}$ | H | H | N |
| 90 | 4-Nonylcyclohexyl | 4 | Cyclohexyl | H | H | CH |
| 91 | 4-Nonylcyclohexyl | 4 | Cyclohexyl | H | H | N |
| 92 | 4-Nonylcyclohexyl | 5 | n-$C_6H_{13}$ | H | H | CH |
| 93 | 4-Nonylcyclohexyl | 5 | Cyclohexyl | H | H | CH |
| 94 | 4-Nonylcyclohexyl | 6 | n-$C_6H_{13}$ | H | H | CH |
| 95 | 4-Nonylcyclohexyl | 6 | Cyclohexyl | H | H | CH |
| 96 | 4-tert.-Butyl-cyclohexyl | 4 | n-$C_3H_7$ | H | H | N |

| | | | | | | |
|---|---|---|---|---|---|---|
| 97 | 4-tert.-Butyl-cyclohexyl | 4 | $n-C_3H_7$ | H | H | CH |
| 98 | 4-tert.-Butyl-cyclohexyl | 4 | $n-C_6H_{13}$ | H | H | N |
| 99 | 4-tert.-Butyl-cyclohexyl | 4 | $n-C_6H_{13}$ | H | H | CH |
| 100 | 4-tert.-Butyl-cyclohexyl | 4 | Cyclohexyl | H | H | N |
| 101 | 4-tert.-Butyl-cyclohexyl | 4 | Cyclohexyl | H | H | CH |
| 102 | 4-tert.-Butyl-cyclohexyl | 5 | $n-C_6H_{13}$ | H | H | CH |
| 103 | 4-tert.-Butyl-cyclohexyl | 5 | Cyclohexyl | H | H | CH |
| 104 | 4-tert.-Butyl-cyclohexyl | 6 | $n-C_6H_{13}$ | H | H | CH |
| 105 | 4-tert.-Butyl-cyclohexyl | 6 | $n-C_6H_{13}$ | H | H | CH |
| 106 | 4-Cyclohexyl-hexyl | 4 | $n-C_3H_7$ | H | H | CH |
| 107 | 4-Cyclohexyl-hexyl | 4 | $n-C_3H_7$ | H | H | N |
| 108 | 4-Cyclohexyl-hexyl | 4 | $n-C_6H_{13}$ | H | H | CH |
| 109 | 4-Cyclohexyl-hexyl | 4 | $n-C_6H_{13}$ | H | H | N |
| 110 | 4-Cyclohexyl-hexyl | 4 | Cyclohexyl | H | H | CH |
| 111 | 4-Cyclohexyl-hexyl | 4 | Cyclohexyl | H | H | N |
| 112 | 4-Cyclohexyl-hexyl | 5 | $n-C_6H_{13}$ | H | H | CH |
| 113 | 4-Cyclohexyl-hexyl | 5 | Cyclohexyl | H | H | CH |
| 114 | 4-Cyclohexyl-hexyl | 6 | $n-C_6H_{13}$ | H | H | CH |
| 115 | 4-Cyclohexyl-hexyl | 6 | Cyclohexyl | H | H | CH |
| 116 | Isobornyl | 4 | $n-C_3H_7$ | H | H | CH |
| 117 | Isobornyl | 4 | $n-C_3H_7$ | H | H | N |
| 118 | Isobornyl | 4 | $n-C_6H_{13}$ | H | H | CH |
| 119 | Isobornyl | 4 | $n-C_6H_{13}$ | H | H | N |
| 120 | Isobornyl | 4 | Cyclohexyl | H | H | CH |
| 121 | Isobornyl | 4 | Cyclohexyl | H | H | N |
| 122 | Isobornyl | 5 | $n-C_6H_{13}$ | H | H | CH |
| 123 | Isobornyl | 5 | Cyclohexyl | H | H | CH |
| 124 | Isobornyl | 6 | $n-C_6H_{13}$ | H | H | CH |
| 125 | Isobornyl | 6 | Cyclohexyl | H | H | CH |
| 126 | Bornyl | 4 | $n-C_3H_7$ | H | H | CH |
| 127 | Bornyl | 4 | $n-C_3H_7$ | H | H | N |
| 128 | Bornyl | 4 | $n-C_3H_7$ | H | $iso-C_3H_7$ | CH |
| 129 | Bornyl | 4 | $n-C_6H_{13}$ | H | H | N |

| | | | | | | |
|---|---|---|---|---|---|---|
| 130 | Bornyl | 4 | n-$C_6H_{13}$ | H | H | CH |
| 131 | Bornyl | 4 | n-$C_6H_{13}$ | H | iso-$C_3H_7$ | CH |
| 132 | Bornyl | 5 | n-$C_3H_7$ | H | H | CH |
| 133 | Bornyl | 5 | n-$C_6H_{13}$ | H | H | CH |
| 134 | Bornyl | 5 | Cyclohexyl | H | H | CH |
| 135 | Bornyl | 6 | n-$C_3H_7$ | H | H | CH |
| 136 | Bornyl | 6 | n-$C_6H_{13}$ | H | H | CH |
| 137 | Bornyl | 6 | Cyclohexyl | H | H | CH |
| 138 | Menthyl | 4 | n-$C_3H_7$ | H | H | CH |
| 139 | Menthyl | 4 | n-$C_3H_7$ | H | H | N |
| 140 | Menthyl | 4 | n-$C_6H_{13}$ | H | H | CH |
| 141 | Menthyl | 4 | n-$C_6H_{13}$ | H | H | N |
| 142 | Menthyl | 4 | Cyclohexyl | H | H | CH |
| 143 | 1,5-Dimethylbicyclo[2.3.1]-octyl | 4 | n-$C_3H_7$ | H | H | CH |
| 144 | 1,5-Dimethylbicyclo[2.3.1]-octyl | 4 | n-$C_3H_7$ | H | H | N |
| 145 | 1,5-Dimethylbicyclo[2.3.1]-octyl | 4 | n-$C_6H_{13}$ | H | H | CH |
| 146 | 1,5-Dimethylbicyclo[2.3.1]-octyl | 4 | n-$C_6H_{13}$ | H | H | N |
| 147 | 1,5-Dimethylbicyclo[2.3.1]-octyl | 4 | Cyclohexyl | H | H | CH |
| 148 | 1,5-Dimethylbicyclo[2.3.1]-octyl | 4 | Cyclohexyl | H | H | N |
| 149 | 1,5-Dimethylbicyclo[2.3.1]-octyl | 4 | Cyclohexyl | H | H | CH |
| 150 | 1,5-Dimethylbicyclo[2.3.1]-octyl | 4 | Cyclohexyl | H | H | N |
| 151 | 1-Adamantyl | 4 | n-$C_6H_{13}$ | H | H | CH |
| 152 | 2-Adamantyl | 4 | n-$C_6H_{13}$ | H | H | CH |
| 153 | 3,3,5-Trimethylcyclohexyl | 4 | n-$C_3H_7$ | H | H | CH |
| 154 | 3,3,5-Trimethylcyclohexyl | 4 | n-$C_3H_7$ | H | H | N |
| 155 | 3,3,5-Trimethylcyclohexyl | 4 | Cyclohexyl | H | H | CH |

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz, u.a. zur Bekämpfung holzzerstörender Pilze, wie Coniophora puteana und Polystictus versicolor, verwendet werden. Darüber hinaus können mit den neuen Wirkstoffen auch Schimmelpilze und holzverbläuende Pilze wie Pullularia pullulans effektiv bekämpft werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse - wie Gurken, Bohnen und Kürbisgewächse -.

Die neuen Verbindungen sind insbesondere geeignet zur Bekämpfung folgender Pflanzenkrankheiten:
Pseudocercosporella herpotrichoides in Getreide
Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,

Venturia inaequalis (Schorf) an Äpfeln,
Septoria nodorum an Weizen,
Botrytis cinerea (Grauschimmel) an Erdbeeren, Reben,
Rhynchosporium secalis und
Fyrenophora teres in Getreide.

Die Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 bis 5 kg Wirkstoff je ha.

Die Wirkstoffe können auch zusammen mit anderen Wirkstoffen, z. B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten.

Fungizide, die mit den neuen Verbindungen kombiniert werden können, sind beispielsweise:
Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol
2,3-Dicyano-1,4-dithiaanthrachinon
2-Thio-1,3-dithio-(4,5,6)-chinoxalin
1-(Butylcabamoyl)-2-benzimidazol-carbaminsäuremethylester
2-Methoxycarbonylamino-benzimidazol
2-(Furyl-(2))-benzimidazol
2-(Thiazolyl-(4))-benzimidazol
N-(1,1,2,2-Tetrachlorethylthio)-tetrahydrophthalimid
N-Trichlormethylthio-tetrahydrophthalimid
N-Trichlormethylthio-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid

N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4 -triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanon
alpha-(2-Chlorphenyl-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glutaramid
Hexachlorbenzol
DL-Methyl-N-(2,6-dimethyl-phenyl)-N-furoyl(2)-alaninat
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoylhydantoin
N-(3,5-Dichlorphenyl)1,2-dimethylcyclopropan-1,2-dicarbonsäureimid
2-Cyano-N-(ethylaminocarbonyl)-2-methoximino)-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol.

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze; Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind:

I. Man vermischt 90 Gewichtsteile der Verbindung 3 mit 100 Gewichtsteilen N-Methylpyrrolidon und erhält eine Lösung, die zur Anwendung in Form kleinster Tropfen geeignet ist.

II. 20 Gewichtsteile der Verbindung 7 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der nach Beispiel 10 erhältlichen Verbindungen werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

IV. 20 Gewichtsteile der Verbindung 11 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und Verrühren der Lösung in Wasser, erhält man eine wäßrige Dispersion.

V. 20 Gewichtsteile der Verbindung 13 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der nach Beispiel 14 erhältlichen Verbindung werden mit 95 Gewichtsteilen feinteiligem Kaolin innig vermischt. Man erhält auf diese Weise ein Stäubemittel, das 5 Gew.-% des Wirkstoffs erhält.

VII. 30 Gewichtsteile der Verbindung 15 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 16 werden mit 30 Teilen Natriumsalz eines Phenolsulfonsäureharnstoff- formaldehyd-kondensates, 2 Teilen Kieselgel und 48 Teilen Wasser innig vermischt. Man erhält eine stabile wäßrige Dispersion.

IX. 20 Teile der Verbindung 17 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-kondensates und 68 Teilen eines paraffinischen Mineralöls innig vermischt. Man erhält eine stabile ölige Dispersion.

Die folgenden Versuche belegen die biologische Wirkung der neuen Verbindungen. Als Vergleichsmittel wurde der bekannte Wirkstoff N-Isopropyl-N-phenyloxyethyl-N'-imidazolylharnstoff verwendet.

**Anwendungsbeispiel 1**

Wirksamkeit gegen Weizenmehltau

Blätter von in Töpfen gewachsenen Weizenkeimlingen der Sorte Frühgold werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ausmaß der Mehltauentwicklung ermittelt.

Das Ergebnis zeigt, daß die Verbindungen 3, 7, 10, 11, 13, 14, 15, 16, 17, 18, 20, 21, bei der Anwendung als 0,025 %-ige Wirkstoffbrühe eine bessere fungizide Wirkung zeigen (beispielsweise 97 %) als der bekannte Wirkstoff (60 %).

**Anwendungsbeispiel 2**

Wirksamkeit gegen Septoria nodorum

Blätter von in Töpfen gewachsenen Weizenkeimlingen der Sorte "Jubilar" werden mit wäßriger Spritzbrühe, die 80 % Wirkstoff und 20 % Emulgiermittel in der Trockensubstanz enthält, bis zur Tropfnässe besprüht. Am folgenden Tag werden die angetrockneten Pflanzen mit einer wäßrigen Sporensuspension von Septoria nodorum infiziert und dann für 7 Tage bei 17 - 19°C und 95 %-iger relativer Luftfeuchtigkeit weiter kultiviert. Das Ausmaß des Pilzbefalls wird dann visuell ermittelt.

Das Ergebnis zeigt, daß die Verbindungen 4, 10, 11, bei der Anwendung als 0,1 %-ige Wirkstoffbrühe eine bessere fungizide Wirkung zeigen (beispielsweise 90 %) als der bekannte Wirkstoff (50 %).

**Patentansprüche**

1. N-Carbomoyl-azole der Formel

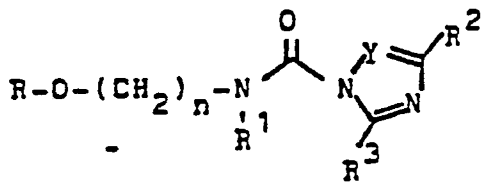

(I),

in der R einen gegebenenfalls alkyl-, alkoxyalkyl-, alkenyl-, cycloalkylsubstituierten Cycloalkyl- oder Bicycloalkylrest oder einen mono- oder bicyclischen Terpenylrest bedeutet, n = 4, 5 oder 6 $R^1$ Alkyl, Alkenyl, Alkoxyalkyl, Alkylthioalkyl, Cycloalkyl oder Cycloalkylalkyl, Alkoxy, Alkenoxy, Alkoxyalkyloxy, Cycloalkyloxy, Cycloalkylalkyloxy jeweils mit bis zu 12 Kohlenstoffatomen oder einen gegebenenfalls durch Halogen, $C_1$ - $C_4$-Alkyl, $C_1$ - $C_4$-Alkoxy, Trifluormethyl, Nitro oder Cyan substituierten Benzylrest bedeutet und

Y CH, C-$CH_3$ oder N bedeutet, und $R^2$ und $R^3$ gleich oder verschieden sind und Wasserstoff oder Alkyl mit 1 bis 5 C-Atomen bedeuten.

2. N-Carbamoyl-azole der Formel I gemäß Anspruch 1,

dadurch gekennzeichnet, daß R Cyclopentyl, Cyclohexyl, Cycloheptyl, Cyclooctyl, Cyclodecyl, Cyclododecyl, 4-Nonylcyclohexyl, 4-Hexylcyclohexyl, 4-Butylcyclohexyl, 4-tert.-Butylcyclohexyl, 4-Cyclohexyl-cyclohexyl, 2-Cyclohexylcyclohexyl, 3-Cyclohexylcyclohexyl, 1,5-Dimethylbicyclo[2.3.1]octyl, 3,3,5-Trimethylcyclohexyl, 2,4-Dimethylcyclohexyl-, 9-Ethyl-bicyclo[3.3.1]non-9-yl, Norbornyl, Menthyl, Bornyl, Isobornyl, Carvenyl, Pinanyl, Caranyl, 5-Norbonen-2-yl, Verbenyl, Isopinocamphenyl, 1-Adamantyl, 2-Adamantyl bedeutet,

$R^1$ Ethyl, n-Propyl, Isopropyl, n-Butyl, Isobutyl, sek.-Butyl, tert.-Butyl, n-Pentyl, 3-Methyl-1-butyl, n-Hexyl, 3,3-Dimethylbutyl-1, n-Heptyl, n-Otyl, 2,4,4-Trimethylpentyl, 2-Ethylhexyl, n-Decyl, n-Dodecyl, Cyclopropylmethyl, Cyclopentyl, Cyclohexyl, 4-Methylcyclo-hexyl, Cyclohexylmethyl, 4-tert.-Butylcyclohexyl, 2-Methoxyethyl, 2-Ethoxymethyl, 2-Propoxyethyl, 2-Butoxyethyl, 3-Methoxypropyl, 3-Ethoxypropyl, 3-Propoxypropyl, 3-Butoxypropyl, Allyl, 2-Buten-1-yl, 2-Penten-1-yl, 2-Octen-1-yl, Benzyl, 4-Methylbenzyl, alpha-Methylbenzyl, alpha-Ethylbenzyl, 2-, 3- und 4-Fluorbenzyl, 2-, 3- und 4-Chlorbenzyl, 4-Brombenzyl, 3- und 4-Trifluormethylbenzyl, 4-tert.-Butyl-benzyl, p-Methoxybenzyl, 2,4-Dichlorbenzyl, Phenylethyl, Ethoxy, n-Propyloxy, Isopropyloxy, n-Butyloxy, n-Hexyloxy, Cyclohexyloxy, 4-Cyclohexyloxybutyloxy, 2-Ethoxyethyloxy bedeuten,

$R^2$ und $R^3$, Wasserstoff, Methyl, Ethyl oder Isopropyl bedeuten,

Y = CH, N oder C-$CH_3$ bedeutet und

n = 4; 5 oder 6 bedeutet.

3. Verfahren zur Herstellung von N-Carbamoyl-azolen der Formel 1 gemäß Anspruch 1, dadurch gekennzeichnet, daß man eine Verbindung der Formel

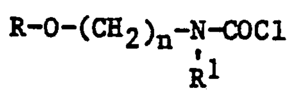

(II)

in der R, $R^1$ und n die im Anspruch 1 angegebene Bedeutungen haben,

a) mit Azolen der Formel

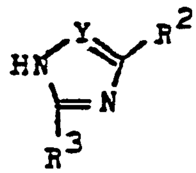

(III),

in der $R^2$, $R^3$ und Y die im Anspruch 1 angegebenen Bedeutungen haben, oder

b) mit deren Metall-Derivaten der Formel

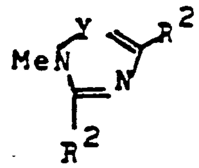

(IV),

in der $R^2$, $R^3$ und Y die im Anspruch 1 angegebenen Bedeutungen haben und Me Lithium, Natrium, Kalium oder 1/2 Calcium bedeutet, oder

c) mit deren Silyl-Derivaten der Formel

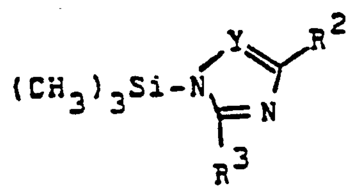

(V),

in der $R^2$, $R^3$ und Y die im Anspruch 1, angegebenen Bedeutungen haben, umsetzt.

4. Verfahren zur Herstellung von N-Carbamoyl-azolen der Formel 1 gemäß Anpsruch 1, <u>dadurch gekennzeichnet</u>, daß man eine Verbindung der Formel

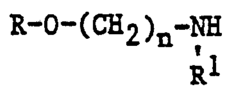

(VI)

in der R, $R^1$ und n die im Anspruch 1 angegebenen Bedeutungen haben mit einem Carbonyl-bisazol der Formel

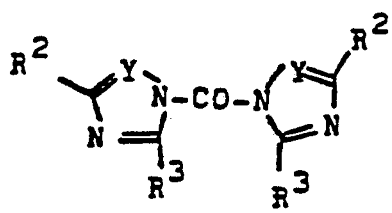

(VII),

in der $R^2$, $R^3$ und Y die im Anspruch 1 angegebenen Bedeutungen haben, umsetzt.

5. Fungizides Mittel, enthaltend ein N-Carbamoyl-azol der Formel I gemäß Anspruch 1.

6. Fungizides Mittel, enthaltend einen inerten Zusatzstoff und ein N-Carbamoyl-azol der Formel

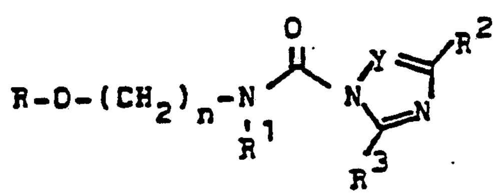

(I),

in der R einen gegebenenfalls alkyl-, alkoxyalkyl-, alkenyl-, cycloalkylsubstituierten Cycloalkyl- oder Bicycloalkylrest oder einen mono- oder bicyclischen Terpenylrest bedeutet, n = 4; 5 oder 6, $R^1$ Alkyl, Alkenyl, Alkoxyalkyl, Alkoxyalkyloxy, Cycloalkyloxy, Cycloalkylalkyloxy jeweils mit bis zu 12 Kohlenstoffatomen oder einen gegebenenfalls durch Halogen, $C_1$-$C_4$-Alkyl, $C_1$-$C_4$-Alkoxy, Trifluormethyl, Nitro oder Cyan substituierten Benzylrest bedeutet und Y CH, C-$CH_3$ oder N bedeutet, und $R^2$ und $R^3$ gleich oder verschieden sind und

Wasserstoff oder Alkyl mit 1 bis 5 C-Atomen bedeuten.

7. Verfahren zur Bekämpfung von Pilzen, dadurch gekennzeichnet, daß man ein N-Carbamoyl-azol der Formel

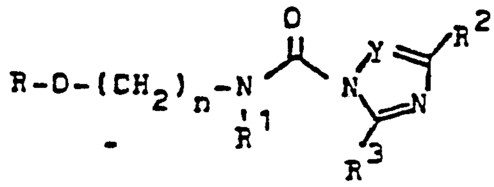

(I),

in der R einen gegebenenfalls alkyl-, alkoxyalkyl-, alkenyl-, cycloalkylsubstituierten Cycloalkyl- oder Bicycloalkylrest oder einen mono- oder bicyclischen Terpenylrest bedeutet, n = 4; 5 oder 6, $R^1$ Alkyl, Alkenyl, Alkoxyalkyl, Alkoxyalkyloxy, Cycloalkyloxy, Cycloalkylalkyloxy jeweils mit bis zu 12 Kohlenstoffatomen oder einen gegebenenfalls durch Halogen, $C_1$-$C_4$-Alkyl, $C_1$-$C_4$-Alkoxy, Trifluormethyl, Nitro oder Cyan substituierten Benzylrest bedeutet und Y CH, C-$CH_3$ oder N bedeutet, und $R^2$ und $R^3$ gleich oder verschieden sind und Wasserstoff oder Alkyl mit 1 bis 5 C-Atomen bedeuten, auf die Pilze oder auf durch Pilzbefall bedrohte Materialien, Flächen, Pflanzen oder Saatgüter einwirken läßt.

8. Verfahren zur Herstellung eines fungiziden Mittels, dadurch gekennzeichnet, daß man ein N-Carbamoyl-azol der Formel

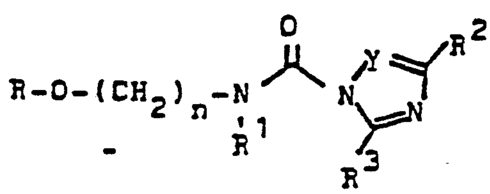

(I),

in der R einen gegebenenfalls alkyl-, alkoxyalkyl-, alkenyl-, cycloalkylsubstituierten Cycloalkyl- oder Bicycloalkylrest oder einen mono- oder bicyclischen Terpenylrest bedeutet, n = 4; 5 oder 6, $R^1$ Alkyl, Alkenyl, Alkoxyalkyl, Alkoxyalkyloxy, Cycloalkyloxy, Cycloalkylalkyloxy jeweils mit bis zu 12 Kohlenstoffatomen oder einen gegebenenfalls durch Halogen, $C_1$-$C_4$-Alkyl, $C_1$-C4-Alkoxy, Trifluormethyl, Nitro oder Cyan substituierten Benzylrest bedeutet und Y CH, C-$CH_3$ oder N bedeutet, und $R^2$ und $R^3$ gleich oder verschieden sind und Wasserstoff oder Alkyl mit 1 bis 5 C-Atomen bedeuten, mit einem festen oder flüssigen Trägerstoff sowie gegebenenfalls mit einem oder mehreren oberflächenaktiven Mitteln mischt.

**Claims**

1. An N-carbamoylazole of the formula

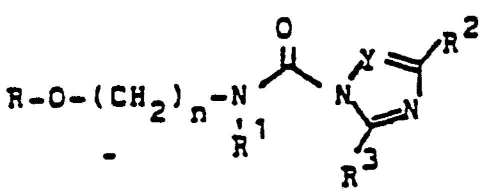

(I)

where R is a cycloalkyl or bicycloalkyl radical which is unsubstituted or substituted by alkyl, alkoxyalkyl, alkenyl or cycloalkyl or is a monocyclic or bicyclic terpenyl radical, n is 4, 5 or 6, $R^1$ is alkyl, alkenyl, alkoxyalkyl, alkylthioalkyl, cycloalkyl or cycloalkylalkyl, alkoxy, alkenyloxy, alkoxyalkoxy, cycloalkoxy or cycloalkylalkoxy, each of not more than 12 carbon atoms, or benzyl which is unsubstituted or substituted by halogen, $C_1$-$C_4$-alkyl, $C_1$-$C_4$-alkoxy, trifluoromethyl, nitro or cyano, and Y is CH, C-$CH_3$ or N, and $R^2$ and $R^3$ are identical or different and are each hydrogen or alkyl of 1 to 5 carbon atoms.

2. An N-carbamoylazole of the formula I as claimed in claim 1, wherein R is cyclopentyl, cyclohexyl, cycloheptyl, cyclooctyl, cyclodecyl, cyclododecyl, 4-nonylcyclohexyl, 4-hexylcyclohexyl, 4-butylcyclohexyl, 4-tert-butylcyclohexyl, 4-cyclohexylcyclohexyl, 2-cyclohexylcyclohexyl, 3-cyclohexylcyclohexyl, 1,5-dimethylbicyclo-[2.3.1]octyl, 3,3,5-trimethylcyclohexyl, 2,4-dimethylcyclohexyl, 9-ethylbicyclo[3.3.1]non-9-yl, norbornyl, menthyl, bornyl, isobornyl, carvenyl, pinanyl, caranyl, 5-norbornen-2-yl, verbenyl, isopinocamphenyl, 1-adamantyl or 2-adamantyl,

$R^1$ is ethyl, n-propyl, isopropyl, n-butyl, isobutyl, sec-butyl, tert-butyl, n-pentyl, 3-methyl-1-butyl, n-hexyl, 3,3-dimethylbut-1-yl, n-heptyl, n-octyl, 2,4,4-trimethylpentyl, 2-ethylhexyl, n-decyl, n-dodecyl, cyclopropylmethyl, cyclopentyl, cyclohexyl, 4-methylcyclohexyl, cyclohexylmethyl, 4-tert-butylcyclo-hexyl, 2-methoxyethyl, 2-ethoxymethyl, 2-proroxyethyl, 2-butoxyethyl, 3-methoxypropyl, 3-ethoxypropyl, 3-pro-poxypropyl, 3-butoxypropyl, allyl, 2-buten-1-yl, 2-penten-1-yl, 2-octen-1-yl, benzyl, 4-methylbenzyl, alpha-methylbenzyl, alpha-ethylbenzyl, 2-, 3- or 4-fluorbenzyl, 2-, 3- or 4-chlorobenzyl, 4-bromobenzyl, 3- or 4-tri-fluormethylbenzyl, 4-tert-butylbenzyl, p-methoxybenzyl, 2,4-dichlorobenzyl, phenylethyl, ethoxy, n-propoxy, iso-propoxy, n-butoxy, n-hexyloxy, cyclohexyloxy, 4-cyclohexyloxybutoxy or 2-ethoxyethoxy,

$R^2$ and $R^3$ are each hydrogen, methyl, ethyl or isopropyl,

Y is CH, N or C-$CH_3$ and

n is 4, 5 or 6.

3. A process for the preparation of an N-cabamoylazole of the formula I as claimed in claim 1, wherein a compound of the formula

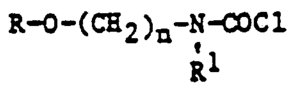

(II)

where R, $R^1$ and n have the meanings stated in claim 1, are reacted

a) with an azole of the formula

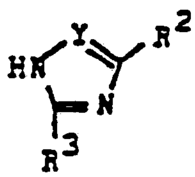

(III)

where $R^2$, $R^3$ and Y have the meanings stated in claim 1, or

b) with one of its metal derivatives of the formula

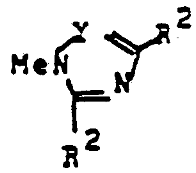

(IV)

where $R^2$, $R^3$ and Y have the meanings stated in claim 1 and Me is lithium, sodium, potassium or 1/2 calcium, or

c) with one of its silyl derivatives of the formula

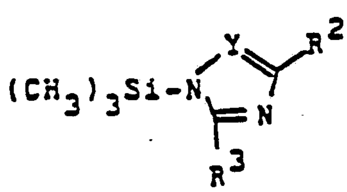

(V)

where $R^2$, $R^3$ and Y have the meanings given in claim 1.

4. A process for the preparation of an N-carbamoylazole of the formula I as claimed in claim 1, wherein a compound of the formula

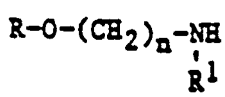

(VI)

where R, $R^1$ and n have the meanings stated in claim 1, is reacted with a carbonylbisazole of the formula

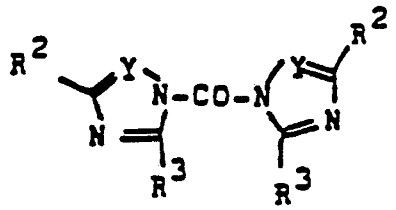

(VII)

where $R^2$, $R^3$ and Y have the meanings stated in claim 1.

5. A fungicide containing an N-carbamoylazole of the formula I as claimed in claim 1.

6. A fungicide containing an inert additive and an N-carbamoylazole of the formula

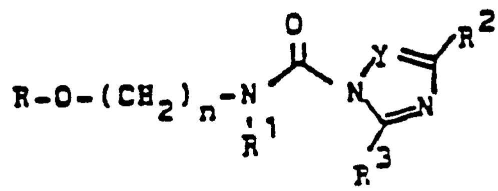

(I)

where R is a cycloalkyl or bicycloalkyl radical which is unsubstituted or substituted by alkyl, alkoxyalkyl, alkenyl or cycloalkyl or is a monocyclic or bicyclic terpenyl radical, n is 4, 5 or 6, $R^1$ is alkyl, alkenyl, alkoxyalkyl, alkoxyalkoxy, cycloalkoxy or cycloalkylalkoxy, each of not more than 12 carbon atoms, or benzyl which is unsubstituted or substituted by halogen, $C_1$-$C_4$-alkyl, $C_1$-$C_4$-alkoxy, trifluoromethyl, nitro or cyano, and Y is CH, $C$-$CH_3$ or N, and $R^2$ and $R^3$ are identical or different and are each hydrogen or alkyl of 1 to 5 carborn atoms.

7. A method for controlling fungi, wherein an N-carbamoylazole of the formula

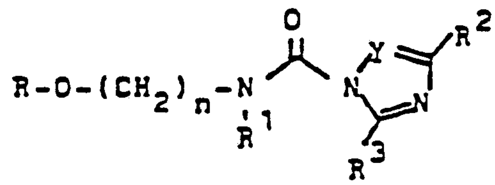

(I)

where R is a cycloalkyl or bicycloalkyl radical which is unsubstituted or substituted by alkyl, alkoxyalkyl, alkenyl or cycloalkyl or is a monocyclic or bicyclic terpenyl radical, n is 4, 5 or 6, $R^1$ is alkyl, alkenyl, alkoxyalkyl, alkoxyalkoxy, cycloalkoxy or cycloalkylalkoxy, each of not more than 12 carbon atoms, or is benzyl which is unsubstituted or substituted by halogen, $C_1$-$C_4$-alkyl, $C_1$-$C_4$-alkoxy, trifluoromethyl, nitro or cyano, and Y is CH, $C$-$CH_3$ or N, and $R^2$ and $R^3$ are identical or different and are each hydrogen or alkyl of 1 to 5 carbon atoms, is allowed to act on the fungi or on materials, areas, plants or seed threatened by fungal attack.

8. A process for the preparation of a fungicide, wherein an N-carbamoylazole of the formula

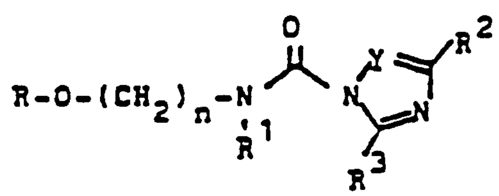

(I)

where R is a cycloalkyl or bicycloalkyl radical which is unsubstituted or substituted by alkyl, alkoxyalkyl, alkenyl, or cycloalkyl or is a monocyclic or bicyclic terpenyl radical, n is 4, 5 or 6, $R^1$ is alkyl, alkenyl, alkoxyalkyl, alkoxyalkoxy, cycloalkoxy or cycloalkylalkoxy, each of not more than 12 carbon atoms, or is benzyl which is unsubstituted or substituted by halogen, $C_1$-$C_4$-alkyl, $C_1$-$C_4$-alkoxy, trifluoromethyl, nitro or cyano, and Y is CH, $C$-$CH_3$ or N, and $R^2$ and $R^3$ are identical or different and are each hydrogen or alkyl of 1 to 5 carbon atoms, is mixed with the solid or liquid carrier and, if required, with one or more surfactants.

**Revendications**

1. N-Carbamoyle-azoles de formule

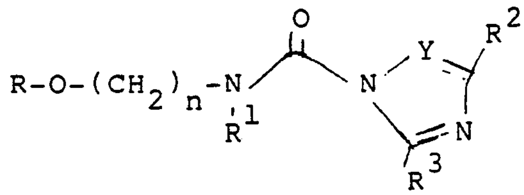 (I),

dans laquelle R représente un reste cycloalkyle ou bicycloalkyle éventuellement substitué par alkyle, alcoxyalkyle, alcényle, cycloalkyle, ou un reste terpényle mono- ou bicyclique, n = 4, 5 ou 6, $R^1$ représente alkyle, alcényle, alkoxyalkyle, alkylthioalkyle, cycloalkyle ou cycloalkylalkyle, alcoxy, alcénoxy, alcoxyalkyloxy, cycloalkyloxy, cycloalkylalkyloxy chacun ayant jusqu'à 12 atomes de carbone ou un reste benzyle eventuellement substitué par halogène, alkyle en $C_1$-$C_4$,alcoxy en $C_1$-$C_4$ trifluorométhyle nitro ou cyano et Y représente CH, C-$CH_3$ ou N et $R^2$ et $R^3$ sont identiques ou différents et représentent hydrogène ou alkyle à 1 à 5 atomes C.

2. N-carbamoyle-azoles de formule I, selon la revendication 1, caractérisés par le fait que R représente cyclopentyle cyclohéxyle, cycloheptyle, cyclooctyle, cyclodécyle, cyclododécyle, 4-nonylcyclohexyle, 4-héxylcyclohéxyle, 4-butylcyclohéxyle, 4-tert.-butylcyclohéxyle, 4-cyclohéxylcyclohéxyle, 2-cyclohexylcyclohéxyle, 3-cyclohexylcyclohéxyle, 1,5-diméthylbicyclo[2.3.1]octyle, 3,3,5-trimethylcyclohexyle, 2,4-diméthylcyclohéxyle-, 9-éthyl-bicyclo[3.3.1]non-9-yle, norbornyle, mentyle, bornyle, isobornyle, carvenyle, pinanyle, caranyle, 5-norbonen-2-yle, verbenyle, isopinocamphényle, 1-adamantyle, 2-adamantyle, $R^1$ représente éthyle, n-propyle, isopropyle, n-butyle, isobutyle, sek.-butyle, tert.-butyle, n-pentyle, 3-méthyl-1-butyle, n-héxyle, 3,3-diméthylbutyle-1, n-heptyle, n-otyle, 2,4,4-triméthylpentyle, 2-éthylhéxyle, n-décyle, n-dodécyl, cyclopropylméthyle, cyclopentyle, cyclohéxyle, 4-méthylcyclohéxyle, cyclohéxylméthyle, 4-tert.-butylcyclohéxyle, 2-méthoxyéthyle, 2-éthoxyméthyle, 2-propoxyéthyle, 2-butoxyéthyle, 3-méthoxypropyle, 3-éthoxypropyle, 3-propoxypropyle, 3-butoxypropyle, allyle, 2-buten-1-yle, 2-penten-1-yle, 2-octen-1-yle, benzyle, 4-méthylbenzyle, α-méthylbenzyle, α-ethylbenzyle, 2-, 3- et 4-fluorobenzyle, 2-, 3- et 4-chlorobenzyle, 4-bromobenzyle, 3- et 4-trifluorométhylbenzyle, 4-tert.-butylbenzyle, p-méthoxybenzyle, 2,4-dichlorobenzyle, phényléthyle, éthoxy, n-propyloxy, isopropyloxy, n-butyloxy, n-héxyloxy, cyclohéxyloxy, 4-cyclohéxyloxybutyloxy, 2-éthoxyéthyloxy

$R^1$ et $R^3$ representent hydrogène, méthyle, éthyle ou isopropyle,

Y = CH, N ou C-$CH_3$ et

n = 4, 5 ou 6.

3. Procédé de préparation de N-carbonyl-azoles de formule I selon la revendication 1, caractérisé par le fait que l'on fait réagir un composé de formule

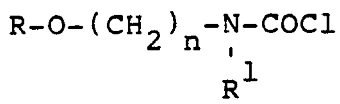 (II),

dans laquelle R, $R^1$ et n ont des significations données dans la revendication 1,

a) avec des azoles de formule

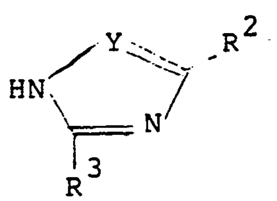 (III),

dans laquelle $R^2$, $R^3$ et Y ont les significations données dans la revendication 1, ou

b) avec leurs dérivés métalliques de formule

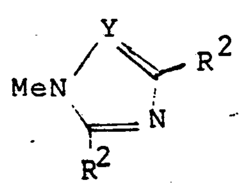 (IV),

dans laquelle $R^2$, $R^3$ et Y ont les significations données dans la revendication 1 et Me représente lithium, sodium, potassium ou 1/2 calcium, ou

c) avec leurs dérivés silyliques de formule

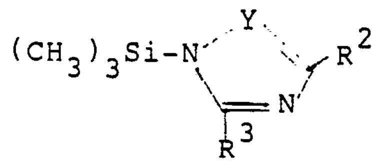 (V),

dans laquelle $R^2$, $R^3$ et Y ont les significations données dans la revendication 1.

4. Procédé de préparation de N-carbamoyl-azoles de formule I selon la revendication 1, caractérisé par le fait que l'on fait réagir un composé de formule

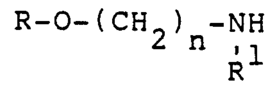 (VI),

dans laquelle R, $R^1$ et n ont les significations données dans la revendication 1 avec un carbonyl-bisazole de formule

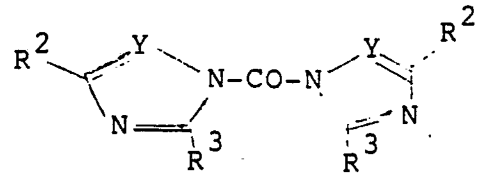 (VII),

dans laquelle $R^2$, $R^3$ et Y ont les significations données dans la revendication 1.

5. Agent fongicide, contenant un N-carbamoyl-azole de formule I selon la revendication 1.

6. Agent fongicide, contenant un additif inerte et un N-carbamoyl-azole de formule

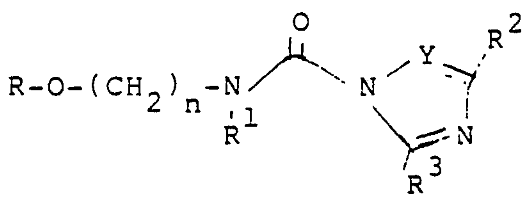 (I),

dans laquelle R représente un reste cycloalkyle ou bicycloalkyle éventuellement substitué par alkyle, alcoxyalkyle, alcényle, cycloalkyle, ou un reste terpényle mono- ou bicyclique, n = 4, 5 ou 6, $R^1$ représente alkyle, alcényle, alkoxyalkyle, alkylthioalkyle, cycloalkyle ou cycloalkylalkyle, alcoxy, alcénoxy, alcoxyalkyloxy, cycloalkyloxy, cycloalkylalkyloxy chacun ayant jusqu'à 12 atomes de carbone ou un reste benzyle eventuellement substitué par halogène, alkyle en $C_1$-$C_4$ alcoxy en $C_1$-$C_4$, trifluorométhyle, nitro, ou cyano et Y represente CH, C-$CH_3$ ou N et $R^2$ et $R^3$ sont identiques ou différents et représentent hydrogène ou alkyle à 1 à 5 atomes C.

7. Procédé de lutte contre les champignons, caractérisé par le fait que l'on fait agir sur le champignon ou les matériaux, surfaces, plantes ou semences menacés par l'attaque de champignons un N-carbamoyl-azole de formule

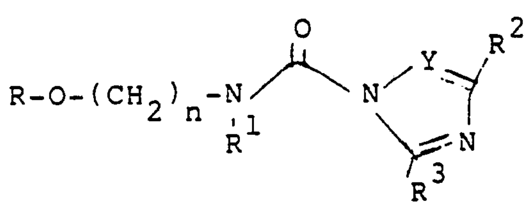 (I),

dans laquelle R représente un reste cycloalkyle ou bicycloalkyle éventuellement substitué par alkyle, alcoxyalkyle, alcényle, cycloalkyle, ou un reste terpényle mono- ou bicyclique, n = 4, 5 ou 6, $R^1$ représente alkyle, alcényle, alkoxyalkyle, alkylthioalkyle, cycloalkyle ou cycloalkylalkyle, alcoxy, alcénoxy, alcoxyalkyloxy, cycloalkyloxy, cycloalkylalkyloxy chacun ayant jusqu'à 12 atomes de carbone ou un reste benzyle eventuellement substitué par halogène, alkyle en $C_1$-$C_4$, alcoxy en $C_1$-$C_4$, trifluorométhyle, nitro, ou cyano et Y represente CH, C-$CH_3$ ou N et R et R sont identiques ou différents et représentent hydrogène ou alkyle à 1 à 5 atomes C.

8. Procédé de préparation d'un agent fongicide, caractérisé par le fait que l'on mélange, avec un véhicule solide ou liquide et éventuellement aussi avec un ou plusieurs agents tensio-actifs, un N-carbamoyl-azole de formule

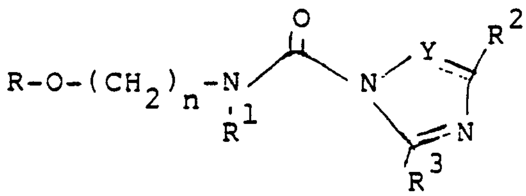

(I),

dans laquelle R représente un reste cycloalkyle ou bicycloalkyle éventuellement substitué par alkyle, alcoxyalkyle, alcényle cycloalkyle, ou un reste terpényle mono- ou bicyclique, n = 4, 5 ou 6, $R^1$ représente alkyle, alcényle, alkoxyalkyle, alkylthioalkyle, cycloalkyle ou cycloalkylalkyle, alcoxy, alcénoxy, alcoxyalkyloxy, cycloalkyloxy, cycloalkylalkyloxy chacun ayant jusqu'à 12 atomes de carbone ou un reste benzyle eventuellement substitué par halogène, alkyle en $C_1$-$C_4$, alcoxy en $C_1$-$C_4$, trifluorométhyle, nitro, ou cyano et Y représente CH, C-$CH_3$ ou N et $R^2$ et $R^3$ sont identiques ou différents et représentent hydrogène ou alkyle à 1 à 5 atomes C.